(19) **Europäisches Patentamt / European Patent Office / Office européen des brevets**

(11) **EP 2 078 523 A1**

(12) **EUROPEAN PATENT APPLICATION**
published in accordance with Art. 153(4) EPC

(43) Date of publication:
**15.07.2009 Bulletin 2009/29**

(21) Application number: **07829727.2**

(22) Date of filing: **12.10.2007**

(51) Int Cl.:
**A61K 31/198** (2006.01)  **A23L 1/305** (2006.01)
**A23L 1/307** (2006.01)  **A61P 1/00** (2006.01)
**A61P 3/04** (2006.01)  **A61P 43/00** (2006.01)

(86) International application number:
**PCT/JP2007/069991**

(87) International publication number:
**WO 2008/044770 (17.04.2008 Gazette 2008/16)**

(84) Designated Contracting States:
**AT BE BG CH CY CZ DE DK EE ES FI FR GB GR HU IE IS IT LI LT LU LV MC MT NL PL PT RO SE SI SK TR**
Designated Extension States:
**AL BA HR MK RS**

(30) Priority: **13.10.2006 JP 2006280713**

(71) Applicant: **Ajinomoto Co., Inc.**
**Tokyo**
**104-8315 (JP)**

(72) Inventors:
• **KONISHI, Atsushi**
**Kawasaki-shi, Kanagawa 210-8681 (JP)**

• **ISHIBASHI, Ikumi**
**Kawasaki-shi, Kanagawa 210-8681 (JP)**
• **KITAHARA, Yoshiro**
**Kawasaki-shi, Kanagawa 210-8681 (JP)**
• **MIURA, Kyoko**
**Kawasaki-shi, Kanagawa 210-8681 (JP)**
• **NARASAKA, Sachiko**
**Kawasaki-shi, Kanagawa 210-8681 (JP)**

(74) Representative: **Nicholls, Kathryn Margaret et al**
**Mewburn Ellis LLP**
**33 Gutter Lane**
**London**
**EC2V 8AS (GB)**

(54) **AGENT FOR SUPPRESSION OF GASTRIC EMPTYING COMPRISING 4-HYDROXYISOLEUCINE**

(57)    The present invention aims to provide a more effective gastric emptying suppressant and food intake suppressant, wherein the solving means are a gastric emptying suppressant containing 4-hydroxyisoleucine or a salt thereof and a food intake suppressant containing 4-hydroxyisoleucine or a salt thereof.

**EP 2 078 523 A1**

**Description**

Technical Field

The present invention relates to an agent for suppressing expulsion of gastric contents from the stomach. Moreover, the present invention relates to an agent for suppressing food intake. The agent is used as a pharmaceutical agent, food and drink and the like.

Background Art

4-Hydroxyisoleucine (HIL) is one type of amino acid contained only in plants belonging to the genus *trigonella*, and particularly contained in large amounts in fenugreek seeds. Fenugreek has long been used as a herb, and is said to provide effects in diseases such as diabetes and the like. In addition, an extract of fenugreek is known to have actions such as hyperglycemia suppressive action, insulin secretagogue action, neutral fats absorption suppressive action and the like (non-patent documents 1-3). Moreover, a report has documented that ingestion of an extract of fenugreek suppresses body weight gain of high-fat diet-fed mouse (non-patent document 4). However, this report describes that administration of 4-hydroxyisoleucine *per se* does not show such action. It has also been reported that 4-hydroxyisoleucine *per se* shows an insulin secretagogue action in vitro (non-patent document 5), and an antidiabetic action in vivo (non-patent document 6).
Under the circumstances, a new use of 4-hydroxyisoleucine is desired.

> non-patent document 1: Z. Madar et al. Eur. J. Clin. Nutr. 42(1): 51-4, 1988
> non-patent document 2: P. Petit et al. Pharmacol. Biochem. Behav. 45(2): 369-74, 1993
> non-patent document 3: R. D. Sharma et al. Eur. J. Clin. Nutr. 44(4): 301-6, 1990
> non-patent document 4: T. Handa et al. Biosci. Biotechnol. Biochem. 69(6): 1186-8, 2005
> non-patent document 5: Y. Sauvaire et al. Diabetes 47(2): 206-10, 1998
> non-patent document 6: C. Broca et al. Am. J. Physiol. 277 (Endocrinol. Metab. 40): E617-E623, 1999

Disclosure of the Invention

Problems to be Solved by the Invention

The present invention aims to provide a novel use of 4-hydroxyisoleucine or a salt thereof, and specifically aims to provide an agent effective for suppressing gastric emptying. Moreover, it aims to provide an agent effective for suppressing food intake.

Means of Solving the Problems

The present inventors have found that 4-hydroxyisoleucine or a salt thereof has a gastric emptying suppressive effect, as well as a food intake suppressive effect, which resulted in the completion of the present invention. Accordingly, the present invention provides the following.

> [1] A gastric emptying suppressant agent, which comprises 4-hydroxyisoleucine or a salt thereof.
> [2] The agent of the above-mentioned [1], which is a food intake suppressant.
> [3] The agent of the above-mentioned [1] or [2], which comprises 4-hydroxyisoleucine or a salt thereof in an amount of 1 to 99 wt%.
> [4] The agent of any of the above-mentioned [1] to [3], wherein a daily dose of 4-hydroxyisoleucine or a salt thereof to an adult is 10 mg to 10 g based on 4-hydroxyisoleucine.
> [5] The agent of any of the above-mentioned [1] to [4], which is to be used in combination with an anorexiant.
> [6] A pharmaceutical composition comprising the agent of any of the above-mentioned [1] to [5].
> [7] A food or drink comprising the agent of any of the above-mentioned [1] to [5].
> [8] A method of suppressing gastric emptying, which comprises administering an effective amount of 4-hydroxyisoleucine or a salt thereof as an active ingredient to a subject of administration.
> [9] The method of the above-mentioned [8], which suppresses food intake.
> [10] The method of the above-mentioned [8] or [9], wherein the effective amount for an adult is 10 mg to 10 g/day based on 4-hydroxyisoleucine.
> [11] The method of any of the above-mentioned [8] to [10], which is used in combination with an anorexiant.
> [13] Use of 4-hydroxyisoleucine or a salt thereof for the production of a gastric emptying suppressant.

[14] The use of the above-mentioned [13], wherein the gastric emptying suppressant is a food intake suppressant.

[15] The use of the above-mentioned [13] or [14], wherein a daily dose of 4-hydroxyisoleucine or a salt thereof for an adult is 10 mg to 10 g based on 4-hydroxyisoleucine.

[16] The use of any of the above-mentioned [13] to [15], which is for a combined use with an anorexiant.

[17] The use of any of the above-mentioned [13] to [16], wherein the gastric emptying suppressant is a pharmaceutical agent or a food or drink.

[18] The use of the above-mentioned [17], wherein the food or drink is a food with health claims or a dietary supplement.

[19] The use of the above-mentioned [18], wherein the food with health claims is a food for specified health uses or a food with nutrient function claims.

[20] A food with an indication that it comprises 4-hydroxyisoleucine or a salt thereof and is used for suppression of gastric emptying.

[21] The food of the above-mentioned [20], wherein the suppression of gastric emptying is suppression of food intake.

[22] The food of the above-mentioned [20] or [21], which is a food with health claims or a dietary supplement.

[23] The food of the above-mentioned [22], wherein the food with health claims is a food for specified health uses or a food with nutrient function claims.

[24] The food of the above-mentioned [20] to [23], which is in the form of ingestion per one dose as a unit, and the unit comprises not less than 5 mg of 4-hydroxyisoleucine.

[25] A commercial package comprising a substance comprising 4-hydroxyisoleucine or a salt thereof, and a written matter containing an explanation relating to use of the substance for suppression of gastric emptying.

[26] The commercial package of the above-mentioned [25], which relates to a pharmaceutical agent.

[27] The commercial package of the above-mentioned [25], which relates to a food.

[28] The commercial package of the above-mentioned [25] to [27], wherein the use for suppression of gastric emptying is use for suppression of food intake.

[29] The commercial package of the above-mentioned [28], which relates to a pharmaceutical agent.

[30] The commercial package of the above-mentioned [28], which relates to a food.


Effect of the Invention

The agent of the present invention has a superior effect of suppressing expulsion of gastric contents from the stomach, or a gastric emptying suppressive effect, as well as a superior food intake suppressive effect. Moreover, since the agent of the present invention contains 4-hydroxyisoleucine or a salt thereof as an active ingredient, it causes an extremely low adverse reaction.


Brief Description of the Drawings

Fig. 1 shows a gastric emptying-suppressive action of 4-hydroxyisoleucine or a salt thereof (HIL).

Fig. 2 shows a food intake-suppressive action of 4-hydroxyisoleucine or a salt thereof (HIL).


Best Mode for Carrying Out the Invention

The present invention provides a gastric emptying suppressant, which comprises 4-hydroxyisoleucine or a salt thereof.

In the present invention, the "gastric emptying" means delivery of gastric contents such as food, digested food and the like in the stomach from the pyloric part to the duodenum. By suppression of gastric emptying, the gastric contents stay in the stomach for a long time. It is known that suppression of gastric emptying suppresses appetite (M. Bickel et al. Int. J. Obes. 28, 211-221, 2004). Therefore, suppression of gastric emptying suppresses further food intake. The gastric emptying suppressant of the present invention has an action to suppress "further food intake". The "further food intake" here means food intake before expulsion of gastric contents from the stomach. That is, since the gastric emptying is suppressed and the gastric contents stay in the stomach for a long time in the present invention, "the time up to further food intake" is prolonged. To "suppress further food intake" means not only to suppress further food intake itself but also suppress appetite, where frequency of food intake decreases and/or the amount of further food intake decreases, thus reducing the total food intake in one day, as compared to non-administration of the gastric emptying suppressant of the present invention. Therefore, the gastric emptying suppressant of the present invention is also useful, for example, for a subject having difficulty in controlling excessive appetite such as binge eating and the like.

In addition, the present invention also provides a food intake suppressant comprising 4-hydroxyisoleucine or a salt thereof.

In the present invention, "suppression of food intake" means suppression of appetite to reduce the amount of food intake.

In the following explanation, unless particularly indicated, the "gastric emptying suppressant" and the "food intake suppressant" are sometimes combined and referred to as "the agent of the present invention". In the present invention, moreover, the "agent" includes not only pharmaceutical preparations (pharmaceutical compositions) but also food and drink (food compositions).

In the present invention, all of L-form, D-form and DL-form of 4-hydroxyisoleucine can be used. Preferred are L-form and DL-form, and more preferred is L-form. Also, 4-hydroxyisoleucine is known to contain two kinds of diastereoisomers ((2S,3R,4S)-4-hydroxyisoleucine and (2R,3R,4S)-4-hydroxyisoleucine) and a lactone form, all of which are usable. Preferred is (2S,3R,4S)-4-hydroxyisoleucine. The present invention also encompasses use of any of them. In addition, 4-hydroxyisoleucine is a concept including a salt thereof and a derivative thereof. The salts and derivatives are also encompassed in the present invention. They are generically referred to as "4-hydroxyisoleucine" in the present specification.

As mentioned above, 4-hydroxyisoleucine in the present invention is a concept also embracing a salt thereof. Examples of the "salt" include a salt with inorganic base, a salt with organic acid and the like. Examples of the salt with inorganic base include alkali metal salts such as sodium, potassium, lithium and the like, alkaline earth metal salts such as calcium, magnesium and the like, and a salt with ammonium salt and the like. Examples of the salt with inorganic acid include salts with hydrohalic acid (hydrochloric acid, hydrobromic acid, hydroiodic acid etc.), sulfuric acid, nitric acid, phosphoric acid and the like. Examples of the salt with organic acid include salts with formic acid, acetic acid, propionic acid, oxalic acid, succinic acid, maleic acid, fumaric acid, citric acid, glutamic acid, aspartic acid and the like. Of these, alkali metal salts such as sodium salt and the like are preferable.

As mentioned above, 4-hydroxyisoleucine in the present invention is a concept also embracing a derivative. Examples of the "derivative" include the derivative described in US 2006/0199853 and the like.

In the present invention, 4-hydroxyisoleucine or a salt thereof may be a commercially available product or may be extracted from a plant belonging to the genus *trigonella* (e.g., seeds of fenugreek, etc.). In the present invention, moreover, 4-hydroxyisoleucine or a salt thereof may be synthesized according to a known chemical method.

The content of 4-hydroxyisoleucine or a salt thereof in the agent of the present invention is preferably 1 - 99 wt%, more preferably 40 - 99 wt%, still more preferably 60 - 99 wt%. The content varies depending on the form of the agent. It is preferable to contain at least not less than 5 mg of 4-hydroxyisoleucine or a salt thereof (as 4-hydroxyisoleucine) in the agent, and the upper limit thereof is preferably 10 g. In this case, the agent does not cause an adverse reaction, but exhibits a superior gastric emptying suppressive effect and also a superior food intake suppressive effect.

The agent of the present invention is useful as a pharmaceutical agent, food, drink and the like. Examples of the administration or ingestion subject thereof include mammals (e.g., human, mouse, rat, hamster, rabbit, cat, dog, bovine, sheep, monkey etc.).

While the mode of administration of the agent of the present invention is not particularly limited, it is preferably oral administration. As the dosage form, general dosage forms for pharmaceutical preparations such as tablet (including sugar-coated tablet, film-coated tablet), pill, capsule, divided powder preparation, suspension, liquid, syrup, gum preparation, drop preparation, powder and the like can be employed. Similar dosage forms can also be employed for foods.

In an attempt to provide an effect of suppressing further food intake and/or enhance food intake suppressive effect, the agent of the present invention can be used in combination with other anorexiants (any of pharmaceutical product and food as long as it has such action). In this event, the agent of the present invention is preferably administered orally by adding to an anorexiant or a preparation or food different from an anorexiant. Usable anorexiant is not particularly limited as long as it is a pharmaceutical agent capable of suppressing appetite by directly or indirectly acting on the appetite center. Examples thereof include centrally-acting appetite suppressive substance, physiologically active peptide and the like. Specifically, mazindol, leptin, glucagon-like peptide 1 (GLP-1), sibutramine, peptide YY (PYY), Type 4 melanocortin receptor agonist, cannabinoid receptor antagonist and the like can be mentioned.

Pharmaceutical agents and foods in such forms are prepared according to a conventional method. When necessary for formulation of preparations, various kinds of pharmacologically acceptable substances for formulation of preparations can be added. The substance for formulation of preparations can be appropriately selected according to the dosage form of the preparation. Examples thereof include excipient, diluent, disintegrant, binder, coating agent, lubricant, gliding agent, lubricant, flavor-improving agent, sweetening agent, solubilizer and the like. Specific examples of the substance for formulation of preparations include magnesium carbonate, titanium dioxide, lactose, mannitol and other saccharides, talc, milk protein, gelatin, starch, cellulose and a derivative thereof, animal and vegetable oils, polyethylene glycol, and solvents such as sterilized water and monovalent or polyvalent alcohol (e.g., glycerol) and the like. Examples of the form of the agent of the present invention include solid, powder, liquid, semi-solid and the like.

The content of 4-hydroxyisoleucine or a salt thereof in the agent of the present invention is preferably 100 mg to 10 g as 4-hydroxyisoleucine.

While the dose of the gastric emptying suppressant or food intake suppressant of the present invention when used as a pharmaceutical agent varies depending on the age, body weight and pathology of the subject patients, dosage form, administration method and the like, the dose of 4-hydroxyisoleucine or a salt thereof per day for an adult (body weight 60 kg) is 10 mg to 10 g based on 4-hydroxyisoleucine. In the case of a regular adult, the dose of 4-hydroxyisoleucine or a salt thereof per day for an adult is preferably 50 mg to 2 g, more preferably 100 mg to 500 mg, based on 4-hydroxyisoleucine. The content of 4-hydroxyisoleucine or a salt thereof in the pharmaceutical agent of the present invention is generally 1 to 99 wt%, preferably 40 to 99 wt%, more preferably 60 to 99 wt%. The above-mentioned daily dose can be administered in one to several portions (e.g., 3 times) before, during or after meal or between meals. It is preferably taken before meal, for example, 0 hr - 2 hr before meal, preferably 0 hr - 1 hr before meal, more preferably 0 hr - 0.5 hr before meal. In addition, the administration period is not particularly limited.

The agent of the present invention can be conveniently used even in the form of a food. The food of the present invention contains 4-hydroxyisoleucine or a salt thereof, and is ingested for the particular purpose of "suppression of gastric emptying" and "suppression of food intake". In addition, the food of the present invention may be a general food including what is called a health food. Furthermore, the food of the present invention can be a food with health claims, a food for specified health uses, a food with nutrient function claims, or further, a dietary supplement (nutrition aid food), which are defined in the food with health claims system by the Ministry of Health, Labour and Welfare.

While the food of the present invention can take the form of the aforementioned preparations, 4-hydroxyisoleucine or a salt thereof may be added to a general food material, seasoning, flavoring agent and the like, which may then be added to drinkable preparation, gum, powder, tablet, granule, jelly and the like, to facilitate intake thereof.

When the food of the present invention is what is called a health food, for example, a packing form wherein not less than 5 mg of 4-hydroxyisoleucine or a salt thereof in powder or granule is packed for ingestion per one dose as a unit, and the like can be mentioned, and when the food is a health drink, not less than 5 mg of 4-hydroxyisoleucine or a salt thereof as 4-hydroxyisoleucine is suspended or dissolved and packed in a bottle and the like for complete ingestion at one time can be mentioned. In the case of such form, the upper limit of 4-hydroxyisoleucine or a salt thereof is preferably 10 g. Here, the form for ingestion per one dose as a unit means a form containing a predetermined amount to be ingested for one dose and, for example, a predetermined amount of drink, candy, chewing gum, jelly, purine, yogurt and the like may be defined by a pack, packaging, bottle etc, and a predetermined amount of granule, powder or slurry foods may be defined by a packaging and the like, or an amount to be ingested for one dose may be indicated on a container and the like.

When the food of the present invention is used for the particular object mentioned above, the amount of 4-hydroxyisoleucine or a salt thereof to be ingested per day by an adult (body weight 60 kg) is preferably 10 mg to 10 g, more preferably 50 mg to 2 g, and still more preferably 100 mg to 500 mg, based on 4-hydroxyisoleucine. While the content of 4-hydroxyisoleucine or a salt thereof in the food of the present invention varies depending on the form of the food, it is generally 1 to 99 wt%, preferably 1 to 50 wt%, more preferably 1 to 30 wt%. When the content of 4-hydroxyisoleucine or a salt thereof in the above-mentioned general foods is within the above-mentioned range, a remarkable effect of suppression of gastric emptying or suppression of food intake can be provided.

The present invention also encompasses a commercial package comprising a substance containing 4-hydroxyisoleucine or a salt thereof and a written matter describing that the substance can or should be used for suppressing gastric emptying. The package can be applied to both a pharmaceutical agent and a food.

In addition, the present invention also encompasses a commercial package comprising a substance containing 4-hydroxyisoleucine or a salt thereof and a written matter describing that the substance can or should be used for suppressing food intake. The package can be applied to both a pharmaceutical agent and a food.

The contents disclosed in any publication cited in the present specification, including patents and patent applications, are hereby incorporated in their entireties by reference, to the extent that they have been disclosed herein.

The present invention is explained in detail in the following by referring to Examples, which are not to be construed as limitative.

Examples

1. Gastric emptying-suppressive action

Male Sprague Dawley rats (12 weeks of age, Charles River Laboratories Japan Inc.) (n=5) were used. After fasting for 20 hours, distilled water for injection (5 mL/kg) was orally administered to a vehicle group and 4-hydroxyisoleucine (500 mg/5 mL/kg) was orally administered to a 4-hydroxyisoleucine administration group. One hour later, a test meal (1.5% methylcellulose, 0.05% phenol red, 1.5 ml) was orally administered. After 90 minutes, the stomach was isolated under an ether anesthesia, the gastric contents were recovered and the remaining phenol red was quantified. A control group was prepared. After fasting for 20 hours, a test meal (1.5% methylcellulose, 0.05% phenol red, 1.5 mL) was orally administered. Immediately after administration of the test meal, the stomach was isolated under an ether anesthesia, the gastric contents were recovered and the remaining phenol red was quantified. The gastric emptying was calculated by the following formula.

$$\text{Gastric emptying (\%)} = (1 - \text{phenol red amount of each individual/average phenol red amount of control group*}) \times 100$$

$$*\text{:test meal alone was administered, } n=2$$

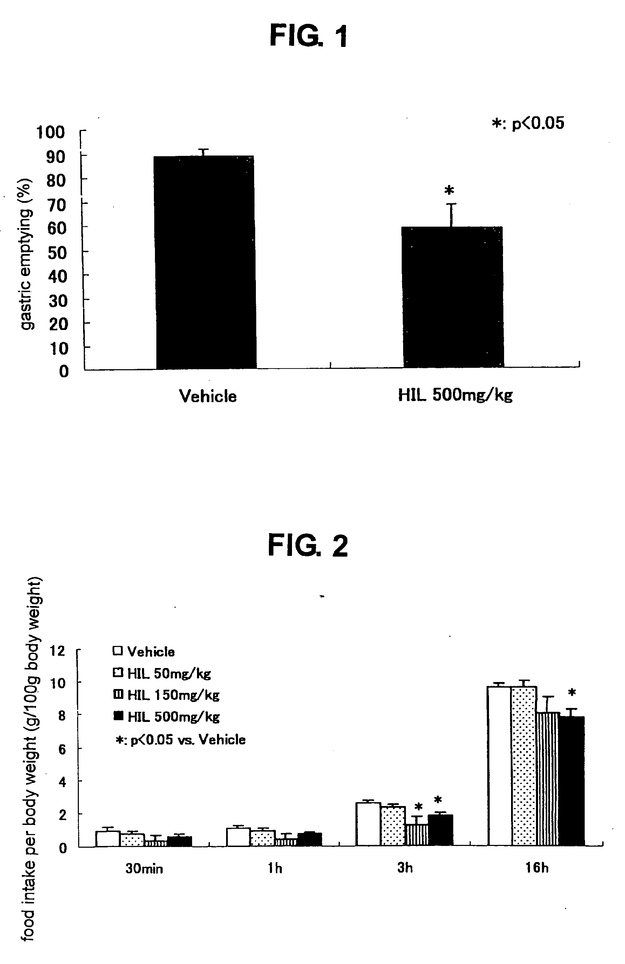

Fig. 1 shows Mean±SE. The difference between the 4-hydroxyisoleucine (HIL) administration group and the distilled water for injection (vehicle) administration group was detected according to Student's t test, and $p < 0.05$ was judged as significant.

As a result, gastric emptying was significantly suppressed by the administration of 4-hydroxyisoleucine (500 mg/kg) as shown in Fig. 1.

2. Food intake suppressive action

Male Wistar rats (7 weeks of age, Charles River Laboratories Japan Inc.) (n=5) were used. After fasting for 9 hours, distilled water for injection (5 mL/kg) or 4-hydroxyisoleucine (50, 150, 500 mg/5 mL/kg) was orally administered at 18:00. Immediately thereafter, feeding was started. The cumulative feed intake was measured at 30 min, 1 hr, 3 hr and 16 hr after the administration.

Fig. 2 shows Mean±SE. The difference between the 4-hydroxyisoleucine administration group and the distilled water for injection (vehicle) administration group was detected according to Student's t test, and $p < 0.05$ was judged as significant.

As a result, food intake was significantly suppressed by the administration of 4-hydroxyisoleucine (500 mg/kg) as shown in Fig. 2.

**Industrial Applicability**

The agent of the present invention has a superior gastric emptying suppressive effect and also has a superior food intake suppressive effect. Moreover, since the agent of the present invention contains 4-hydroxyisoleucine or a salt thereof as an active ingredient, it causes only extremely low adverse reaction.

This application is based on a patent application No. 2006-280713 filed in Japan, the contents of which are incorporated in full herein by this reference.

**Claims**

1. A gastric emptying suppressant agent, which comprises 4-hydroxyisoleucine or a salt thereof.

2. The agent of claim 1, which is a food intake suppressant.

3. The agent of claim 1 or 2, which comprises 4-hydroxyisoleucine or a salt thereof in an amount of 1 to 99 wt%.

4. The agent of any one of claims 1 to 3, wherein a daily dose of 4-hydroxyisoleucine or a salt thereof to an adult is 10 mg to 10 g based on 4-hydroxyisoleucine.

5. The agent of any one of claims 1 to 4, which is to be used in combination with an anorexiant.

6. A pharmaceutical composition or a food or drink comprising the agent of any one of claims 1 to 5.

7. A method of suppressing gastric emptying, which comprises administering an effective amount of 4-hydroxyisoleucine or a salt thereof as an active ingredient to a subject of administration.

8. The method of claim 7, which suppresses food intake.

9.  The method of claim 7 or 8, wherein the effective amount for an adult is 10 mg - 10 g/day based on 4-hydroxyisoleucine.

10. The method of any one of claims 7 to 9, which is used in combination with an anorexiant.

11. Use of 4-hydroxyisoleucine or a salt thereof for the production of a gastric emptying suppressant.

12. The use of claim 11, wherein the gastric emptying suppressant is a food intake suppressant.

13. The use of claim 11 or 12, wherein a daily dose of 4-hydroxyisoleucine or a salt thereof for an adult is 10 mg - 10 g based on 4-hydroxyisoleucine.

14. The use of any one of claims 11 to 13, which is for a combined use with an anorexiant.

15. The use of any one of claims 11 to 14, wherein the gastric emptying suppressant is a pharmaceutical agent or a food or drink.

EP 2 078 523 A1

# FIG. 1

# FIG. 2

## INTERNATIONAL SEARCH REPORT

| | International application No. |
|---|---|
| | PCT/JP2007/069991 |

A. CLASSIFICATION OF SUBJECT MATTER
*A61K31/198*(2006.01)i, *A23L1/305*(2006.01)i, *A23L1/307*(2006.01)i, *A61P1/00*
(2006.01)i, *A61P3/04*(2006.01)i, *A61P43/00*(2006.01)i

According to International Patent Classification (IPC) or to both national classification and IPC

B. FIELDS SEARCHED

Minimum documentation searched (classification system followed by classification symbols)
A61K31/198, A23L1/305, A23L1/307, A61P1/00, A61P3/04, A61P43/00

Documentation searched other than minimum documentation to the extent that such documents are included in the fields searched
Jitsuyo Shinan Koho        1922-1996   Jitsuyo Shinan Toroku Koho   1996-2007
Kokai Jitsuyo Shinan Koho  1971-2007   Toroku Jitsuyo Shinan Koho   1994-2007

Electronic data base consulted during the international search (name of data base and, where practicable, search terms used)
BIOSIS(STN), FSTA(STN), CAplus(STN), EMBASE(STN), MEDLINE(STN),
REGISTRY(STN),
PubMed, JMEDPlus(JDream2), JST7580(JDream2), JSTPlus(JDream2)

C. DOCUMENTS CONSIDERED TO BE RELEVANT

| Category* | Citation of document, with indication, where appropriate, of the relevant passages | Relevant to claim No. |
|---|---|---|
| X<br>Y | JP 2006-089449 A  (TSI CHINA CO., LTD.),<br>06 April, 2006 (06.04.06),<br>Full text; particularly, Claim 1; Par. Nos.<br>[0001], [0007], [0008]; examples<br>(Family: none) | 1-6,11-15<br>5,14 |
| X<br>Y | Koji YAMAGUCHI, "Fenugreek Ekisu (Promilin) no<br>Atarashii Seiri Kassei", Food Processing and<br>ingredients (2004), Vol.39, No.8, pages 51 to 53 | 1-6,11-15<br>5,14 |
| Y | WO 2005/049006 A1  (AJINOMOTO CO., INC, JP),<br>02 June, 2005 (02.06.05),<br>Full text; particularly, Claim 2<br>& EP 1685833 A1          & US 2006/205633 A1 | 5,14 |

☒ Further documents are listed in the continuation of Box C.   ☐ See patent family annex.

| * Special categories of cited documents: | "T" later document published after the international filing date or priority date and not in conflict with the application but cited to understand the principle or theory underlying the invention |
|---|---|
| "A" document defining the general state of the art which is not considered   to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search<br>    06 December, 2007 (06.12.07) | Date of mailing of the international search report<br>    18 December, 2007 (18.12.07) |
|---|---|
| Name and mailing address of the ISA/<br>    Japanese Patent Office | Authorized officer |
| Facsimile No. | Telephone No. |

Form PCT/ISA/210 (second sheet) (April 2007)

9

**INTERNATIONAL SEARCH REPORT**

International application No.

PCT/JP2007/069991

C (Continuation).    DOCUMENTS CONSIDERED TO BE RELEVANT

| Category* | Citation of document, with indication, where appropriate, of the relevant passages | Relevant to claim No. |
|---|---|---|
| Y | Japanese Journal of Clinical Medicine, Vol.53· 1995 Nen Tokubetsugo Himansho, Nippon Rinshosha, 1995, pages 481 to 486 | 5,14 |
| A | JONES KL, Berry M, Kong MF, Kwiatek MA, Samsom M, Horowitz M., Hyperglycemia attenuates the gastrokinetic effect of erythromycin and affects the perception of postprandial hunger in normal subjects., Diabetes Care.,1999 Feb, Vol.22, No.2,pages339-344 | 1-6,11-15 |

Form PCT/ISA/210 (continuation of second sheet) (April 2007)

**INTERNATIONAL SEARCH REPORT**

| International application No. |
| --- |
| PCT/JP2007/069991 |

**Box No. II      Observations where certain claims were found unsearchable (Continuation of item 2 of first sheet)**

This international search report has not been established in respect of certain claims under Article 17(2)(a) for the following reasons:

1. ☒ Claims Nos.: 7-10
   because they relate to subject matter not required to be searched by this Authority, namely:
   Claims 7 to 10 pertain to methods for treatment of the human body by surgery or therapy.

2. ☐ Claims Nos.:
   because they relate to parts of the international application that do not comply with the prescribed requirements to such an extent that no meaningful international search can be carried out, specifically:

3. ☐ Claims Nos.:
   because they are dependent claims and are not drafted in accordance with the second and third sentences of Rule 6.4(a).

**Box No. III      Observations where unity of invention is lacking (Continuation of item 3 of first sheet)**

This International Searching Authority found multiple inventions in this international application, as follows:

1. ☐ As all required additional search fees were timely paid by the applicant, this international search report covers all searchable claims.

2. ☐ As all searchable claims could be searched without effort justifying additional fees, this Authority did not invite payment of additional fees.

3. ☐ As only some of the required additional search fees were timely paid by the applicant, this international search report covers only those claims for which fees were paid, specifically claims Nos.:

4. ☐ No required additional search fees were timely paid by the applicant.   Consequently, this international search report is restricted to the invention first mentioned in the claims; it is covered by claims Nos.:

**Remark on Protest**
the

☐ The additional search fees were accompanied by the applicant's protest and, where applicable, payment of a protest fee.

☐ The additional search fees were accompanied by the applicant's protest but the applicable protest fee was not paid within the time limit specified in the invitation.

☐ No protest accompanied the payment of additional search fees.

Form PCT/ISA/210 (continuation of first sheet (2)) (April 2007)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20060199853 A

- JP 2006280713 A

**Non-patent literature cited in the description**

- **Z. Madar et al.** *Eur. J. Clin. Nutr.,* 1988, vol. 42 (1), 51-4
- **P. Petit et al.** *Pharmacol. Biochem. Behav.,* 1993, vol. 45 (2), 369-74
- **R. D. Sharma et al.** *Eur. J. Clin. Nutr.,* 1990, vol. 44 (4), 301-6
- **T. Handa et al.** *Biosci. Biotechnol. Biochem.,* 2005, vol. 69 (6), 1186-8

- **Y. Sauvaire et al.** *Diabetes,* 1998, vol. 47 (2), 206-10
- **C. Broca et al.** Endocrinol. Metab. *Am. J. Physiol.,* 1999, vol. 277 (40), E617-E623
- **M. Bickel et al.** *Int. J. Obes.,* 2004, vol. 28, 211-221